# EUROPÄISCHE PATENTSCHRIFT

(11) **EP 0 658 544 B1**
(45) Veröffentlichungstag und Bekanntmachung des Hinweises auf die Patenterteilung: **22.10.1997**
(21) Anmeldenummer: 94118928.4
(22) Anmeldetag: 01.12.1994
(51) Int. Cl.: C07C 323/09

(54) **Verbessertes Verfahren zur Herstellung von 2,2'-Dinitrodiphenyldisulfid**
Improved process for the preparation of 2,2'-dinitrodiphenyl disulfide
Procédé amélioré pour la préparation de 2,2'-dinitrodiphényl disulfide

(30) Priorität: 14.12.1993 DE 4342619
(43) Veröffentlichungstag der Anmeldung: 21.06.1995
(73) Patentinhaber: BAYER AG, 51368 Leverkusen (DE)
(72) Erfinder: Hagedorn, Ferdinand, Dr., D-51381 Leverkusen (DE); Fiege, Helmut, Dr., D-51373 Leverkusen (DE); Söllner, Robert, Dr., D-51373 Leverkusen (DE); Helm, Rudolf, Dr., D-51469 Bergisch Gladbach (DE)

(56) Entgegenhaltungen:
- DE-A- 2 204 726
- GB-A- 1 498 410
- GAZZETTA CHIMICA ITALIANA, Bd.110, Nr.5-6, 1980 Seiten 301 - 303 P. BATTISTONI ET AL
- SYNTHESIS, Nr.8, 1981 Seiten 637 - 638 M. KODOMARI ET AL
- CHEMICAL ABSTRACTS, vol. 114, no. 5, 1991, Columbus, Ohio, US; abstract no. 42024p

## Beschreibung

Die vorliegende Erfindung betrifft ein Verfahren zur Herstellung von 2,2'-Dinitrodiphenyldisulfid durch Umsetzung von 2-Chlornitrobenzol mit wäßriger Alkalidisulfid-Lösung, bei dem ein reineres Produkt als bei bekannten Verfahren in sehr guten Ausbeuten erhalten wird.

Die Herstellung von 2,2'-Dinitrodiphenyldisulfid aus 2-Chlornitrobenzol und Natriumdisulfid wird in der Literatur vielfach beschrieben, wobei unterschiedliche Reaktionsmedien für die Umsetzung genannt werden, so z.B. Alkohole (Helv. Chim. Acta 663 [1929] oder Dimethylformamid, N-Methyl-pyrrolidinon und N-Methylcaprolactam (DE-OS 2 204 726). Die Ausbeuten bei der Verwendung von Alkoholen als Lösungsmittel sind im allgemeinen mäßig (68-86 % der Theorie). Mit den obengenannten cyclischen und offenkettigen Carbonsäureamiden werden zwar Ausbeuten bis zu 94 % erhalten, jedoch sind diese speziellen Lösungsmittel so wertvoll, daß sie beim Einsatz in den beschriebenen Mengen in technisch aufwendiger Weise abgetrennt und für die Wiederverwendung aufbereitet werden müssen.

Die Verwendung von Phasentransferkatalysatoren gemaß GB-PS 1 498 410 ergibt Ausbeuten von nur knapp 85 %. Die EP-OS 156 769 beschreibt die Reaktion von 2-Chlornitrobenzol mit Natriumdisulfid in Wasser und in Gegenwart eines nichtionischen und/oder eines anionischen Tensids. Die hierbei benötigte Tensid-Menge ist relativ groß, und zur Entfernung des Tensids aus dem Reaktionsprodukt benötigt man große Mengen von Auswaschmitteln (Wasser oder Lösungsmittel). Ferner tritt bei dieser Arbeitsweise während der Reaktion die Bildung von Klumpen und zähflüssigen Massen auf, die nur mit speziellen Rührvorrichtungen, z.B. einem Dismembrator, handhabbar sind. Das in diesen Klumpen und zähflüssigen Massen enthaltene 2-Chlornitrobenzol reagiert nur sehr langsam bis zur vollständigen Umsetzung ab. Das nach dieser Reaktion isolierte 2,2'-Dinitrodiphenyldisulfid enthält noch Nebenprodukte (laut Beispiel 1 der EP-OS 156 769 hat das Produkt einen Schmelzpunkt von 182-184°C statt 196°C, wie in der Literatur angegeben; laut Beispiel 2 enthält das Produkt ca. 5 % Nebenprodukte).

Es bestand daher die Aufgabe, ein verbessertes Herstellungsverfahren für 2,2'-Dinitrodiphenyldisulfid zu finden, das mit hoher Ausbeute zu einem Produkt mit hoher Reinheit führt und bei dem sich während der Reaktion keine schwer rührbaren, zähflüssigen Massen oder Klumpen bilden.

Es wurde nun ein Verfahren zur Herstellung von 2,2'-Dinitrodiphenyldisulfid durch Umsetzung von 2-Chlornitrobenzol mit wäßriger Alkalidisulfid-Lösung in Gegenwart von Phasentransferkatalysatoren gefunden, das dadurch gekennzeichnet ist, daß man die Reaktion in zusätzlicher Gegenwart eines organischen Lösungsmittels durchführt.

Als Phasentransferkatalysatoren kommen beispielsweise quaternäre Ammonium-und Phosphoniumsalze der Formeln (I) und (II) in Frage 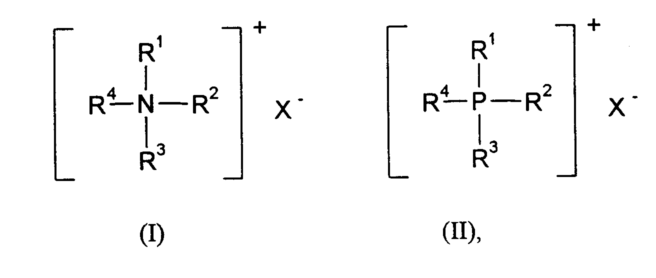 in denen
- R¹ bis R⁴: gleich oder verschieden sind und jeweils für eine C₁-C₁₆-Alkylgruppe, die gegebenenfalls mit einer Hydroxygruppe substituiert sein kann, eine C₆-C₁₀-Arylgruppe, eine C₇-C₁₁-Aralkylgruppe oder einer C₅-C₇-Cycloalkylgruppe stehen, wobei zwei der Reste R¹ bis R⁴ gemeinsam auch einen Ring mit 5 bis 7 C-Atomen bilden können und
- X: für ein Halogen, einen Bisulfat-Rest oder eine Hydroxygruppe steht.

Vorzugsweise sind R¹ bis R⁴ gleich oder verschieden und stehen jeweils für Methyl, Ethyl, n-Propyl, i-Propyl, n-Butyl, i-Butyl, t-Butyl oder Benzyl. Vorzugsweise steht X für Chlor oder Brom.

Besonders bevorzugte Phasentransferkatalysatoren sind Tetra-n-butyl-ammoniumbromid und Benzyltriethylammoniumchlorid, die besonders gut zugänglich sind.

Der Phasentransferkatalysator kann beispielsweise in einer Menge von 0,001 bis 0,2 Moläquivalenten, bezogen auf 2-Chlornitrobenzol, eingesetzt werden. Vorzugsweise liegt diese Menge bei 0,01 bis 0,1 Moläquivalenten.

Als organische Lösungsmittel kommen beispielsweise dipolare, aprotische Lösungsmittel in Frage. Bevorzugt sind dabei N-Methylpyrrolidinon, N-Methylcaprolactam, Dimethylformamid und Dimethylacetamid.

Dipolare, aprotische Lösungsmittel kann man beispielsweise in Mengen von 10 bis 20 Gew.-%, bezogen auf 2-Chlornitrobenzol, einsetzen. Dann ist eine Wiedergewinnung und Aufarbeitung aus wirtschaftlichen Gründen im allgemeinen nicht mehr erforderlich.

Als organische Lösungsmittel kommen beispielsweise auch Alkohole in Frage. Bevorzugt sind aliphatische Alkohole und Etheralkohole mit 1 bis 8 C-Atomen. Besonders bevorzugt sind Methanol, Ethanol, Isopropanol und Isopropanol/Wasser-Gemische mit z.B. 50 bis 95 Gew.-% Isopropanolanteil.

Als organische Lösungsmittel kommen beispielsweise auch mit Wasser vermischbare Ketone in Frage, insbesondere Aceton.

Alkohole und mit Wasser mischbare Ketone, die im Rahmen der Erfindung verwendet werden können, besitzen bei Raumtemperatur ein schlechtes Lösungsvermögen für das herzustellende 2,2'-Dinitrodiphenyldisulfid, sie lösen dagegen 2-Chlornitrobenzol sehr gut. Diese Lösungsmittel sind mit Wasser gut mischbar und behindern bei der Aufarbeitung und Isolierung des Endproduktes nicht das Auswaschen des bei der Reaktion entstehenden Salzes.

Alkohole und mit Wasser mischbare Ketone können z.B. in solchen Mengen eingesetzt werden, daß eine gute Rührbarkeit des Reaktionsgemisches gewährleistet ist. Diese Menge kann daher in weiten Grenzen variiert werden. Häufig benötigt man, um eine gute Rührbarkeit zu erreichen, nur 50 bis 100 Gew.-%, bezogen auf 2-Chlornitrobenzol. Dabei erzielt man eine gute Raumausbeute.

Die Durchführung des Verfahrens gemäß der Erfindung kann z.B. so erfolgen, daß man 2-Chlornitrobenzol in dem gewählten Lösungsmittel mit dem Phasentransferkatalysator vorlegt, dieses Gemisch unter Rühren auf die gewünschte Reaktionstemperatur erhitzt und dann die Alkalidisulfid-Lösung zutropft.

Bei einer besonderen Ausführungsform des erfindungsgemäßen Verfahrens legt man nur einen Bruchteil, z.B. 5 bis 20 Gew.-% des umzusetzenden 2-Chlornitrobenzols mit dem organischen Lösungsmittel und dem Phasentransferkatalysator vor und dosiert dann zeitgleich, aber getrennt voneinander, die Hauptmenge des 2-Chlornitrobenzols und die Alkalisulfidlösung in das Reaktionsgemisch.

Die Dauer der Zugabe der Reaktionspartner richtet sich im wesentlichen nach den technischen Möglichkeiten der Abführung der Reaktionswärme. Bei einer Dauer von 2 bis 4 Stunden für die Zugabe und 1 bis 4 Stunden für die Nachreaktion kann man Ausbeuten bis zu 98 % d.Th. erzielen.

Das erfindungsgemäße Verfahren wird gewöhnlich drucklos durchgeführt. Grundsätzlich kann man auch bei erhöhtem Druck arbeiten, ohne daß nachteilige Auswirkungen auf das Ergebnis auftreten.

Die Reaktionstemperatur kann beim Einsatz von Alkoholen oder mit Wasser mischbaren Ketonen beispielsweise zwischen 20°C und dem Siedepunkt des Lösungsmittels bei Normaldruck liegen. Vorteilhafterweise arbeitet man in siedendem Lösungsmittel, da dann das Rückflußsieden zur Abführung der Reaktionswärme genutzt werden kann. Bei dipolaren, aprotischen Lösungsmitteln kann die Reaktionstemperatur beispielsweise zwischen 20 und 100°C betragen.

2-Chlornitrobenzol und Alkalidisulfid können beispielsweise im Molverhältnis 1,7 bis 3:1 eingesetzt werden. Vorzugsweise beträgt dieses Verhältnis 1,8 bis 2,0:1.

Die wäßrige Alkalidisulfid-Lösung kann beispielsweise mit einer Konzentration von 2 bis 4 mol Alkalidisulfid pro Liter Lösung, bevorzugt 2,7 bis 3,3 mol/l eingesetzt werden. Die Lösung kann auch in erhitzem Zustand zugegeben werden.

Die Aufarbeitung des Reaktionsgemisches kann beispielsweise so erfolgen, daß man nach dem Abkühlen des Gemisches auf Raumtemperatur das rohe, kristalline Reaktionsprodukt durch Filtrieren, Absaugen oder Zentrifugieren von der Mutterlauge trennt, durch Nachwaschen mit Wasser von verbleibendem Salz befreit und, falls erforderlich, durch Nachwaschen mit einem gekühltem Alkohol, z.B. Methanol oder Isopropanol, nachreinigt. Das Endprodukt wird vorzugsweise wasserfeucht isoliert und aufbewahrt, da es in getrockneter Form unter gewissen Umständen zu Staubexplosionen neigen kann.

2,2'-Dinitrodiphenyldisulfid ist ein Zwischenprodukt zur Herstellung von Farbstoffen, Pflanzenschutzmitteln, Pharmazeutika und Kautschukchemikalien.

Das erfindungsgemäße Verfahren weist die überraschenden Vorteile auf, daß die Reaktion wesentlich selektiver, schneller, ohne die Ausbildung von ruhrtechnisch schwer zu beherrschenden Agglomeraten und mit hohen Ausbeuten durchgeführt werden kann.

Mit dem erfindungsgemäßen Verfahren erhält man 2,2'-Dinitrodiphenyldisulfid im allgemeinen in Ausbeuten zwischen 94 und 98 % und in Reinheiten von 98,5 bis 99,5 %.

Im folgenden wird das erfindungsgemäße Verfahren beispielhaft erläutert.

### Beispiele

### Beispiel 1

In einem Sulfierbecher wurden 98,5 g 2-Chlornitrobenzol, 156 ml N-Methylpyrrolidinon und 20,0 g Tetra-n-butylammoniumbromid vorgelegt und unter Rühren auf 90°C erhitzt. Bei dieser Temperatur wurden gleichzeitig, aber getrennt voneinander, a) eine Lösung aus 416,3 g Natriumsulfid (Na₂S x 3H₂O) und 102,5 g Schwefel in 1175 ml Wasser und b) 886,3 g aufgeschmolzenes 2-Chlornitrobenzol innerhalb von 2 Stunden in das Reaktionsgemisch zudosiert.

Nach Beendigung der Zugabe wurde das Gemisch noch eine Stunde bei 90°C nachgerührt, dann auf Raumtemperatur abgekühlt und rohes 2,2'-Dinitrodiphenyldisulfid durch Filtration abgetrennt. Die gelbe Substanz wurde auf dem Filter mit Wasser chloridfrei gewaschen. So wurden nach dem Trocknen 961,5 g 2,2'-Dinitrodiphenyldisulfid mit einem Gehalt von 98,4 Gew.-% (gemäß HPLC-Analyse) erhalten, was einer Ausbeute von 98,2 % d.Th. entsprach. Durch Waschen des Produkts auf dem Filter mit kaltem Isopropanol und Wasser konnte seine Reinheit auf über 99,5 Gew.-% gesteigert werden.

### Beispiel 2

In einem Rührkolben wurden 225 g 2-Chlornitrobenzol, 166 g eines Gemisches aus 85 Gew.-% Isopropanol und 15 Gew.-% Wasser und 3,2 g Tetra-n-butylammoniumbromid vorgelegt und dann unter Rühren auf 70°C erhitzt. Bei dieser Temperatur wurden innerhalb von 4 Stunden 250 ml einer Lösung aus 99,8 g Natriumsulfid (Na₂S x 3H₂O), 25,0 g Schwefel und 180 ml Wasser zugetropft. Das Gemisch wurde abschließend noch 4 Stunden bei 80°C nachgerührt, danach auf 70°C abgekühlt und rohes 2,2'-Dinitrodiphenyldisulfid durch Filtration abgetrennt. Das gelbe Produkt wurde auf der Nutsche mit Isopropanol und Wasser gewaschen bis es chloridfrei war. So wurden nach dem Trocknen 205,2 g eines 99,5 gew.-%igen Produktes erhalten, was einer Ausbeute von 93 % d.Th. entsprach.

Bei einem Vergleichsversuch entsprechend Beispiel 2, der in Abwesenheit von Tetra-n-butylammoniumbromid durchgeführt wurde, wurden 202,9 g 2,2'-Dinitrodiphenyldisulfid mit einem Gehalt von 97 Gew.-% erhalten. Die Ausbeute betrug 89 % d.Th. Das Produkt war wegen zu starker Verunreinigung mit nicht abtrennbaren Nebenprodukten für eine Verwendung als Zwischenprodukt nicht mehr geeignet.

### Beispiel 3

Es wurde verfahren wie in Beispiel 2, jedoch wurde als Lösungsmittel 166 g Methanol und 4,8 g Tetra-n-butylammoniumbromid und 250 ml einer Natriumdisulfidlösung eingesetzt, die hergestellt worden war aus 99,8 g Na₂S x 3H₂O, 25 g Schwefel und 180 ml Wasser. Die Zugabe erfolgte innerhalb von 3,5 Stunden und entsprach der Arbeitsweise von Beispiel 2. Es wurden 205,9 g 2,2'-Dinitrodiphenyldisulfid mit einem Gehalt von über 99,5 % in einer Ausbeute von 93 % d. Th. erhalten.

## Patentansprüche

1. Verfahren zur Herstellung von 2,2'-Dinitrodiphenyldisulfid durch Umsetzung von 2-Chlornitrobenzol mit wäßriger Alkalidisulfid-Lösung in Gegenwart von Phasentransferkatalysatoren, dadurch gekennzeichnet, daß man die Reaktion in zusätzlicher Gegenwart eines organischen Lösungsmittels durchführt.

2. Verfahren nach Anspruch 1, dadurch gekennzeichnet, daß man als Phasentransferkatalysator quaternäre Ammonium- oder Phosphoniumsalze einsetzt.

3. Verfahren nach Ansprüchen 1 bis 2, dadurch gekennzeichnet, daß man 0,001 bis 0,2 Moläquivalente Phasentransferkatalysator, bezogen auf 2-Chlornitrobenzol, einsetzte.

4. Verfahren nach Ansprüchen 1 bis 3, dadurch gekennzeichnet, daß man dipolare, aprotische Lösungsmittel in Mengen von 10 bis 20 Gew.-%, bezogen auf 2-Chlornitrobenzol, einsetzt.

5. Verfahren nach Ansprüchen 1 bis 3, dadurch gekennzeichnet, daß man Alkohole oder mit Wasser mischbare Ketone in Mengen von 50 bis 100 Gew.-%, bezogen auf 2-Chlornitrobenzol, einsetzt.

6. Verfahren nach Ansprüchen 1 bis 5, dadurch gekennzeichnet, daß man die Reaktion beim Einsatz von Alkoholen oder mit Wasser mischbaren Ketonen bei Temperaturen im Bereich von 20°C bis zum Siedepunkt des Lösungsmittels bei Normaldruck und beim Einsatz von dipolaren, aprotischen Lösungsmitteln bei Temperaturen zwischen 20 und 100°C durchführt.

7. Verfahren nach Ansprüchen 1 bis 6, dadurch gekennzeichnet, daß man 2-Chlornitrobenzol, Lösungsmittel und Phasentransferkatalysator vorlegt, dieses Gemisch unter Rühren auf die gewünschte Temperatur erhitzt und dann die Alkalidisulfid-Lösung zutropft.

8. Verfahren nach Ansprüchen 1 bis 6, dadurch gekennzeichnet, daß man 5 bis 20 Gew.-% des umzusetzenden 2-Chlornitrobenzols zusammen mit dem organischen Lösungsmittel und dem Phasentransferkatalysator vorlegt und dann gleichzeitig, aber getrennt voneinander, die restliche Menge 2-Chlornitrobenzol und die Alkalisulfid-Lösung zudosiert.

9. Verfahren nach Ansprüchen 1 bis 8, dadurch gekennzeichnet, daß man 2-Chlornitrobenzol und Alkalidisulfid im Molverhältnis von 1,7 bis 3:1 einsetzt.

10. Verfahren nach Ansprüchen 1 bis 9, dadurch gekennzeichnet, daß man die wäßrige Alkalisulfid-Lösung mit einer Konzentration von 2 bis 4 Mol pro Liter Lösung einsetzt.

## Claims

1. Process for preparing 2,2'-dinitrodiphenyl disulphide by reaction of 2-chloronitrobenzene with an aqueous alkali metal disulphide solution in the presence of phase transfer catalysts, characterized in that the reaction is carried out in the additional presence of an organic solvent.

2. Process according to Claim 1, characterized in that quaternary ammonium or phosphonium salts are used as phase transfer catalyst.

3. Process according to either of Claims 1 and 2, characterized in that from 0.001 to 0.2 molar equivalent of phase transfer catalyst, based on 2-chloronitrobenzene, is used.

4. Process according to any of Claims 1 to 3, characterized in that dipolar, aprotic solvents are used in amounts of from 10 to 20% by weight, based on 2-chloronitrobenzene.

5. Process according to any of Claims 1 to 3, characterized in that alcohols or water-miscible ketones are used in amounts of from 50 to 100% by weight, based on 2-chloronitrobenzene.

6. Process according to any of Claims 1 to 5, characterized in that when using alcohols or water-miscible ketones the reaction is carried out at temperatures in the range from 20°C to the boiling point of the solvent at atmospheric pressure and when using dipolar, aprotic solvents the reaction is carried out at temperatures between 20 and 100°C.

7. Process according to any of Claims 1 to 6, characterized in that 2-chloronitrobenzene, solvent and phase transfer catalyst are initially charged, this mixture is heated while stirring to the desired temperature and the alkali metal disulphide solution is then added dropwise.

8. Process according to any of Claims 1 to 6, characterized in that from 5 to 20% by weight of the 2-chloronitrobenzene to be reacted are initially charged together with the organic solvent and the phase transfer catalyst, and the remaining amount of 2-chloronitrobenzene and the alkali metal sulphide solution are then metered in simultaneously but separately from one another.

9. Process according to any of Claims 1 to 8, characterized in that 2-chloronitrobenzene and alkali metal disulphide are used in a molar ratio of from 1.7 to 3:1.

10. Process according to any of Claims 1 to 9, characterized in that the aqueous alkali metal sulphide solution is used in a concentration of from 2 to 4 mol per litre of solution.

## Revendications

1. Procédé de préparation du disulfure de 2,2'-dinitrodiphényle par réaction du 2-chloronitrobenzène avec une solution aqueuse de disulfure alcalin en présence de catalyseurs de transfert de phase, caractérisé en ce que l'on conduit la réaction en présence également d'un solvant organique.

2. Procédé selon la revendication 1, caractérisé en ce que l'on utilise comme catalyseurs de transfert de phase des sels d'ammonium ou de phosphonium quaternaire.

3. Procédé selon les revendications 1 et 2, caractérisé en ce que l'on utilise 0,001 à 0,2 équivalent molaire de catalyseur de transfert de phase, par rapport au 2-chloronitrobenzène.

4. Procédé selon les revendications 1 à 3, caractérisé en ce que l'on utilise des solvants aprotiques dipolaires en des quantités de 10 à 20% en masse, par rapport au 2-chloronitrobenzène.

5. Procédé selon les revendications 1 à 3, caractérisé en ce que l'on utilise des alcools ou des cétones miscibles à l'eau en des quantités de 50 à 100% en masse, par rapport au 2-chloronitrobenzène.

6. Procédé selon les revendications 1 à 5, caractérisé en ce que l'on conduit la réaction en utilisant des alcools ou des cétones miscibles à l'eau à des températures dans le domaine de 20°C au point d'ébullition du solvant à la pression normale et en utilisant des solvants aprotiques dipolaires à des températures comprises entre 20 et 100°C.

7. Procédé selon les revendications 1 à 6, caractérisé en ce que l'on dispose au préalable le 2-chloronitrobenzène, le solvant et le catalyseur de transfert de phase, on chauffe ce mélange à la température voulue sous agitation puis on ajoute goutte à goutte la solution de disulfure alcalin.

8. Procédé selon les revendications 1 à 6, caractérisé en ce que l'on dispose au préalable 5 à 20% en masse du 2-chloronitrobenzène à faire réagir, en même temps que le solvant organique et le catalyseur de transfert de phase, puis on ajoute pendant la même durée, mais séparément l'une de l'autre, la quantité restante de 2-chloronitrobenzène et la solution de sulfure alcalin.

9. Procédé selon les revendications 1 à 8, caractérisé en ce que l'on utilise le 2-chloronitrobenzène et le disulfure alcalin dans un rapport molaire de 1,7 à 3 :1.

10. Procédé selon les revendications 1 à 9, caractérisé en ce que l'on utilise la solution aqueuse de sulfure alcalin à une concentration de 2 à 4 moles par litre de solution.
